# EUROPEAN PATENT APPLICATION

(11) **EP 2 835 102 A1**
(43) Date of publication of application: **11.02.2015**
(21) Application number: 13179513.0
(22) Date of filing: 07.08.2013
(51) Int. Cl.: A61B 6/00

(54) **Method and system for detecting a portable direct radiographic panel in a multi-stand radiographic exposure room**

(71) Applicant: Agfa Healthcare, 2640 Mortsel (BE)
(72) Inventor: Govaerts, Wim, 2640 Mortsel (BE); Lievens, Geert, 2640 Mortsel (BE)
(74) Representative: Verbrugghe, Anne Marie L.

(57) **Abstract**

The invention relates to a method for detecting the presence of a portable direct radiographic panel in a medical imaging system comprising radiographic exposure stands, each stand and each panel being provided with an NFC tag. The method comprises positioning a panel in an exposure stand such that the NFC tags of the panel and the stand are in each other's operating range, detecting the presence of the panel in the exposure stand by a communication between these NFC tags and communicating the presence of the panel in the exposure stand to the medical imaging system.

## Description

### Field of the invention :

The present invention relates to a method and a system for detecting a portable direct radiographic panel in a medical imaging system comprising multiple radiographic exposure units.

The portable direct radiographic panel will hereinafter often be referred to as DR Panel.

More in particular the invention relates to a method and system for conveniently and operator-friendly detecting the position of the direct radiographic panel in the multiple exposure units of the medical imaging system and to perform a check whether that position corresponds with the position as set forth in the worklist for the forthcoming radiographic exposure.

### Background of the invention.

It is known that radiographic illumination or exposure has important applications in medical imaging, whereby the medical advantages for the patient largely exceed the small risk of damage resulting from such radiographic illumination.

In earlier days radiographic exposures mostly made use of film based on silver halide technology as image capturing medium.

Since a number of years the so-called computed radiography technique has gained wide market acceptance. This technology makes use of a radiographic panel that does not use silver halide technology as the light capturing medium, but uses stimulable phosphors.

This method is described amongst others in detail in the Handbook of Medical Imaging, (ed. R.V. Matter et al., SPIE Press, Bellingham, 2000).

During recent years, radiographic exposures increasingly make use of direct digital radiographic techniques, known as DR (Direct Radiography).

This method is increasingly used as alternative for film-based imaging techniques, as well as for the panels based on the use of stimulable phosphor-technologies, as described supra.

In this digital radiographic method the radiographic exposure energy is captured pixelwise in a radiographycally sensitive panel, and hereupon is converted to electronic image data by means of electronic components. Hereupon the information is read out imagewise and displayed on a suitable monitor for diagnostic purposes by a radiologist.

One of the driving forces behind the success of direct digital radiography is the ability to rapidly visualise the radiographic images and to efficiently and simply communicate over datanetworks to one or more sites for analysis and remote diagnosis by a radiologist or other medical expert. The delays that are characteristic for the development, packaging and physical transport of radiographic films are avoided by the above methods. Also the difficulties arising from the scanning of developed films and the corresponding loss in resolution is avoided by the above techniques.

The advantage of direct radiographic systems over computed radiographic systems, based on stimulable phosphors, is that no read-out (in a digitizer) of the latently captured radiographic image needs to take place. On the contrary, the digital radiographic image promptly or directly can be read for the purpose of evaluating the image from a diagnostic point of view. This diagnosis can take place at a local or remote workstation.

At the beginning the first direct radiographic panels were integrated in the overall radiographic imaging system. The wiring was designed such that minimal trouble to the radiographic operator was caused hereby when the radiographic direct panel was placed for exposure of a body part of a patient.

More recently portable direct radiographic panels have been introduced to the market place. These panels make use of an on-board battery and communicate with the radiographic control panel or workstation, as well as with the datacapturing apparatus and the display components in a tethered or wireless manner.

The latter aspects resulted in a wide acceptance of such portable wireless panels by the marketplace and ensures their practical use in a fully digital radiographic exposure system.

In a hospital or medical diagnosis center, these panels can be used as well in a completely newly installed radiographic imaging system or in a so-called retrofit situation. The term retrofit should be understood as directed to an existing radiographic system, that previously made use of radiographic films or stimulable phosphor plates, and whereby the latter registration media have been replaced by a direct radiographic capturing medium, a so-called direct radiographic or DR panel, without the need to replace the workstation or the radiographic source itself.

The advantage of such a retrofit radiographic system as compared to a completely newly installed radiographic system, is its lower investment cost, as part of the already installed radiographic system can be re-used.

Although portability and wireless communication of the radiographic registration medium clearly is an advantage when portable and wireless DR panels are used, these features also are characterized by the occurrence of problems under practical circumstances of use.

In particular problems occur when such DR Panels are used in a medical imaging system comprising multiple radiographic exposure units or stands.

Such system will hereinafter be referred to as a multi-stand radiographic exposure room or as a medical imaging system comprising one or more radiographic exposure stands.

A medical imaging system comprising multiple radiographic exposure units or stands may comprise for example on the one hand a mobile radiographic exposure unit, and on the other hand a fixed-position exposure unit. The latter exposure unit may further comprise for example a wall and/or a table stand.

The same DR panel may be used in any of these exposure units.

The problem arises when multiple radiographic exposure units are comprised in a medical imgaging system.

It that case it is of vital importance that for each radiographic exposure the DR Panel is correctly positioned, this means that it is positioned in that specific radiographic exposure unit of the medical imaging system where the patient is positioned and that such position corresponds to the positions as set forth for the patient and the DR panel in the radiological worklist.

In such a multi-stand radiographic exposure room, it may happen that by accident the DR panel is placed in one exposure stand, e.g. the wall stand, whereas the patient is positioned in the table stand, or that both the panel and patient are positioned in the same stand, but that such stand is not in conformity with the stand as set forth in the radiological worklist prescribed for the radiological exposure.

Clearly in such a case, the patient might be exposed to radiographic illumination, but no radiographic image will be detected by the DR Panel, as it is ill-positioned. This then results in a re-take of the radiographic exposure, resulting not only in waste of time and effort, but also in needless harmfull double radiographic exposure for the patient.

Without a specific method that enables to reduce to an absolute minimum the probability of ill-positioning the DR Panel, there remains an enhanced risk for an incorrect exposure of a patient, resulting in retakes. On its turn, this results in a number of complaints, confusion, and a loss of time and efforts.

### Problem to be solved

The aim and purpose of the invention is to avoid the abovementioned problems by providing a convenient, automated and user-friendly method and system for the detection of the correct position of a DR Panel in a multi-stand radiographic exposure room.

### Summary of the invention.

The abovementioned aspects are realised by means of the method and the medical imaging system as described in the independent claims.

Specific features of preferred embodiments of the invention are set forth in the dependent claims.

Further advantages and embodiments of the present invention are clarified in the description that follows.

### Description of the invention:

The present invention relates to a method for detecting the presence of a portable direct radiographic panel in a medical imaging system comprising one or more radiographic exposure stands. In such medical imaging system, each radiographic exposure stand and each radiographic panel are provided with an NFC tag. The method then comprises the following steps :
○ positioning the radiographic panel in the radiographic exposure stand such that the NFC tags of the panel and the stand are in each other's operating range;
○ detecting the presence of the radiographic panel in the radiographic exposure stand by a communication between the NFC tag of the panel and the NFC tag of the radiographic exposure stand;
○ communicating the presence of the radiographic panel in the radiographic exposure stand to the medical imaging system.

Also, the invention relates to a medical imaging system comprising one or more radiographic exposure stands and one or more portable direct radiographic panels, comprising:
○ NFC tags provided on each radiographic exposure stand and on each portable direct radiographic panel;
○ means for positioning the radiographic panel in the radiographic exposure stand such that the NFC tag of the panel and the NFC tag of the stand are in each other's operating range;
○ means for detecting the presence of the panel in the radiographic exposure stand by a communication between the NFC tag of the panel and the NFC tag of the radiographic exposure;
○ means for communicating the presence of the radiographic panel in the radiographic exposure stand to the medical imaging system.

### Description of preferred embodiments :

A preferred embodiment of the invention comprises the step of controlling whether for a radiographic exposure for which the exposure conditions are set forth in a radiological worklist, the radiographic exposure stand wherein the presence of the radiographic panel is detected, conforms to the radiographic exposure stand specified in said radiological worklist.

A further preferred embodiment of the invention comprises the step of selecting for a radiographic exposure, when the presence of a radiographic panel in more than one radiographic exposure stand has been detected and the radiological exposure conditions are set forth in a radiological worklist, the radiographic exposure stand specified in said radiological worklist for the radiographic exposure, provided the presence of a radiographic panel in the selected exposure stand has been detected.

Preferably the presence of the radiographic panel in the radiographic exposure stand is communicated to a radiographic workstation of the medical imaging system. An example of such radiographic workstation is the apparatus marketed by Agfa Healthcare N.V. under the brand name NX.

According to a further preferred embodiment of the invention the NFC tag provided on each portable direct radiographic panel is an active NFC tag, and the NFC tag provided on each radiographic exposure stand of the medical imaging system is a passive NFC tag, the communication between the NFC tags being triggered by moving the active NFC tag towards the passive NFC tag such that the latter is within the operational range of the former. In a still further preferred embodiment of the invention, the communication is triggered by the active NFC tag pulling a response to occur from the passive NFC tag provided on the radiographic exposure stand.

The presence of the portable direct radiographic panel in the radiographic exposure stand may be communicated to the medical imaging system by the portable panel by means of a wired ethernet or a wireless short-range radio-wave transmission.

According to a further preferred embodiment of the invention, when the conformity between the presence of the portable direct radiographic panel in the radiographic exposure stand with the radiographic exposure stand as specified in the radiographic worklist is not established, a warning is given to the radiographic operator.

Further, upon the radiographic exposure having taken place, the radiographic image and metadata stored in the direct radiographic panel are transmitted to the medical imaging system.

Further, the medical imaging system according to the invention Defurther comprises means for controlling whether for a radiographic exposure for which the exposure conditions are set forth in a radiological worklist, the radiographic exposure stand wherein the presence of the radiographic panel is detected, conforms to the radiographic exposure stand specified in said radiological worklist.

According to a further preferred embodiment, the medical imaging system further comprises means for selecting, when the presence of a radiographic panel in more than one radiographic exposure stand is detected and the radiological exposure conditions are set forth in a radiological worklist, the radiographic exposure stand specified in said radiological worklist for the radiographic exposure, provided the presence of a radiographic panel in the selected exposure stand has been detected.

In a further preferred embodiment, in the medical imaging system according to the invention, the NFC tag provided on each portable direct radiographic panel is an active tag, and the NFC tag provided on each radiographic exposure unit is a passive tag.

The medical imaging system according to the invention further preferably comprises a radiographic workstation, such as Agfa Healthcare's NX apparatus, and wherein the portable direct radiographic panel and the workstation further comprise means for wireless communication by means of short-range radio waves, preferably through WIFI.

The medical imaging system further may be provided with a radiological workstation provided with means for receiving the communication of the presence of the radiographic panel in a radiographic exposure stand, and for performing the control or selection as set forth supra.

In a still further preferred embodiment of the medical imaging system according to the invention, each radiographic exposure unit comprises at least two NFC tags for determining, apart from the presence, the orientation of the radiographic panel in the radiographic exposure unit.

### Detailed description of the invention :

### Retrofit system:

The method of the present invention is applicable as well in a radiographic exposure system that is newly installed in a medical care centre such as a hospital, or it may be applied in a retrofit situation as described earlier in this text.

### Communication between DR panel and medical imaging system:

Once the presence of the radiographic panel in one of the radiographic exposure units has been detected, this should be communicated to the medical imaging system.

Such communication may be performed by transmitting the presence in a given exposure stand to a radiological workstation, comprised in the medical imaging system.

This workstation may be connected wirelessly or through a wired connection with the portable radiographic panel. A wireless connection is preferred, provided the radiographic panel is a wireless-enabled panel. In that case the radiological workstation and the panel are provided with means for wireless communication, such as a wireless communication processor.

The wireless communication preferably is performed by means of short-range radio waves.

The wireless communication module or processor of the radiographic panel can be used for wirelessly transmitting the spatial positon of the radiographic panel to the radiological workstation, but is may also be used to wirelessly transmit the radiographic image and meta-data stored in the direct radiographic panel to the medical imaging system, more in particular to the radiological workstation comprised in such system.

Such wireless communication module is a means known for the person skilled in the art. Such module has been described e.g. in the US patents of Fuji Photo Film, Inc., Japan, Nr. US 7 829 859 and US 8 116 599. The patent first mentioned describes how the portable radiographic panel transmits the digital image data stored therein over such wireless communication processor to the radiological workstation or radiographic console by means of a transceiver of the radiographic Panel. The UWB (Ultra Wide Band) protocol is mentioned as an example of such wireless communication protocol. Such UWB Protocol is characterised by a substantial reduction of energy-consumption, and by enhanced communication speed, as compared to other wireless communication techniques.

The other US patent, US 8 116 599, describes the conversion to wireless communication signals of the image data by the wireless communication unit according to one of the following existing wireless communciation protocols : UWB, Bluetooth, Zigbee, HiSWANa (High Speed Wireless Access Network type a), HiperLAN, Wireless 1394, Wireless USB, and finally Wireless LAN, infrared (irDA), NFC (Near Field Communication), IO-Homecontrol.

Preferably use is made of a wireless communication protocol working according to the IEEE 802.11 standard.

In such a case, the direct radiographic panel communicates by means of a short-range radio or infrared connection over the wireless network with the radiographic workstation by means of any of the above communication protocols. Generally a short-range radioconnection is preferred over an infrared connection, as the first mentioned connection operates in an omnidirectional manner, whereas for an infrared connection, as it is an optical connection method, a direct optical path should be created between the transmitter and the receiver of the signals.

The wireless LAN Network can make use of a number of various wireless network protocols and mechanisms. Preferably use is made of the wireless IEEE 802.11 g or IEEE 802.11 n interface (WIFI) standard.

One can also make use of the IEEE 802.11 b standard, whereby in a point-to-point configuration (1 point to various points), one acces point (the wireless entry point) through a multidirectional antenna communicates with other clients that are within the range of the central access point.

The one access point may be the modality or radiological workstation, and the other clients are the various portable radiographic panels.

So as to realise such wireless connection, preferably such WIFI connection, with the workstation, the processor has at its disposal on the direct radiographic panel an antenna driver and a chip technology that enables such short-range radioconnection.

### NFC Tag:

The term NFC tag as used in the present description should be understood as an electronic chip, designed specifically for being used according to the NFC set of standards. More in particular an NFC tag is essentially a small microchip containing a small amount of memory attached to an antenna/aerial which can store a small amount of information for transfer to another electronic device according to the NFC standard.

The information on the NFC tag is usually stored in a specific data format (NDEF - NFC data exchange format) so that it can be reliably read by electronic, e.g. mobile devices.

The NFC tags to be used in the method and system of the present invention preferably are no standard tags, as those won't work directly onto metal surface. To that end on-metal NFC tags should be selected, which are available e.g. from RapidNFC, London, U.K.

The NFC tags of the radiographic exposure unit may be installed on the bucky of such unit, either the wall or the table stand.

As set forth supra in a preferred embodiment of the method according to the invention, the exposure stands, e.g. the table and wall bucky may be provided with a passive NFC tag. The advantage of using such passive NFC tag at this position, is that no charging by a battery is required, and that these tags can be quite easily installed.

Given the fact that the range of an NFC tag is quite limited, there also is no risk for false positive detections. So the situation of a panel being detected as present in the bucky whereas in reality the panel is not present there, cannot arise.

On the DR Panel on the contrary, an active NFC tag is used. This has the advantage that the wireless (WIFI) settings of the radiological workstation may be transmitted to the panel through such active NFC tag.

This aspect is described in a co-pending patent application of the same applicant, filed on even date herewith.

### NFC:

The term NFC as used in the present description should be understood as a set of standards for mobile devices such a smartphones and similar devices to establish radio communication with each other by touching them together or bringing them into close proximity, usually no more than a few inches.

NFC standards cover communication protocols and data exchange formats, and are based on existing radio-frequency identification (RFID) standards.

### Link to the HIS/RIS/ Radiological Worklist :

According to a preferred embodiment of the present invention as set forth above, the method further comprises the step of controlling whether for a radiographic exposure for which the exposure conditions are set forth in a radiological worklist, the radiographic exposure stand wherein the presence of the radiographic panel is detected, conforms to the radiographic exposure stand specified in said radiological worklist.

If the result of this conformity check is OK, the operator will proceed to the radiographic exposure.

According to a still further preferred embodiment, in case the conformity between the so identified direct radiographic panel and the direct radiographic panel as set forth in the worklist of the radiographic work station has not been established, a warning is given to the operator. Such warning may comprise a pop-up on the display of the radiographic workstation, optionally including an acoustic or other form of alarm.

In such a case, a manual intervention of the operator is required : he can either adapt the worklist by selecting another DR Panel and/or another exposure unit for the forthcoming exposure or he may reposition the DR panel in the exposure unit designated in the radiological work list for the forthcoming radiographic exposure.

The worklist of the planned radiographic exposures is usually displayed on the screen of the workstation during the various radiographic exposures that are planned for a given time-frame and for a given radiographic exposure room or unit.

Such worklist is part of or comprised within the Radiological Information System (RIS) of the hospital or medical care organisation and is communicated to the work station. Such communication may e.g. comprise the radiographic operator of the radiographic exposure unit concerned to navigate in the Hospital Information System (HIS) to the specific RIS data, and visualising on the screen or display of the radiographic work station such worklist.

The radiographic worklist usually comprises one or more of the following information:
- identity of the patients to be radiographed (name or other personal attributes),
- object to be radiographed (arm, knee, hand, or other body part),
- stand (wall or bucky), as well as
- the digital radiographic panel to be used for the radiographic exposure, and - optionally - the exposure parameters.

### Unique Identification of DR Panel:

Each direct radiographic panel has a unique identification number or other form of identification. Such identification is allocated to the panel at the time of manufacturing the panel or at the time of marketing of the direct radiographic panel.

The abovementioned unique identification code or number of the direct radiographic panel may comprise or consist of a unique serial- or manufacturing-number, or, in an alternate embodiment, may comprise or consist of the fixed or variable IP address, MAC addres or some sort of Unique Identifier allocated to the DR Panel.

The wireless communication module of the direct radiographic panel uses through the wireless communication protocol with the radiographic workstation this unique identification code to distinguish this DR Panel in an unambiguous manner from the other DR panels, and to identify same as such.

### Transmission of image data to the workstation :

In a next step, namely after the radiographic exposure has taken place, the radiographic image data are sent to the radiographic workstation from the DR Panel for visualisation and diagnostic evaluation on the monitor by a radiologist.

### DR Panel in its docking station:

In a radiographic exposure room the various direct radiographic panels mostly are placed in their respective docking stations. The docking station is the place where the direct radiographic panel is positioned when it is not used for a radiographic exposure : through such docking station the DR Panel recharges its on-board battery.

## Claims

1. A method for detecting the presence of a portable direct radiographic panel in a medical imaging system comprising one or more radiographic exposure stands, each radiographic exposure stand and each radiographic panel being provided with an NFC tag, comprising the following steps :
○ positioning a radiographic panel in a radiographic exposure stand such that the NFC tags of the panel and the stand are in each other's operating range;
○ detecting the presence of the radiographic panel in the radiographic exposure stand by a communication between the NFC tag of the panel and the NFC tag of the radiographic exposure;
○ communicating the presence of the radiographic panel in the radiographic exposure stand to the medical imaging system.

2. Method according to claim 1, further comprising the step of controlling whether for a radiographic exposure for which the exposure conditions are set forth in a radiological worklist, the radiographic exposure stand wherein the presence of the radiographic panel is detected, conforms to the radiographic exposure stand specified in said radiological worklist.

3. Method according to claim 1, further comprising the step of selecting for a radiographic exposure, when the presence of a radiographic panel in more than one radiographic exposure stand has been detected and the radiological exposure conditions are set forth in a radiological worklist, the radiographic exposure stand specified in said radiological worklist for the radiographic exposure, provided the presence of a radiographic panel in the selected exposure stand has been detected.

4. Method according to any of the preceding claims, wherein the presence of the radiographic panel in the radiographic exposure stand is communicated to a radiographic workstation of the medical imaging system.

5. Method according to any of the preceding claims, wherein the NFC tag provided on each portable direct radiographic panel is an active NFC tag, and the NFC tag provided on each radiographic exposure stand of the medical imaging system is a passive NFC tag, the communication between the NFC tags being triggered by moving the active NFC tag towards the passive NFC tag such that the latter is within the operational range of the former.

6. Method according to claim 5, wherein the communication is triggered by the active NFC tag pulling a response to occur from the passive NFC tag provided on the radiographic exposure stand.

7. Method according to any of the preceding claims, wherein the presence of the portable direct radiographic panel in the radiographic exposure stand is communicated to the medical imaging system by the portable panel by means of a wired ethernet or a wireless short-range radio-wave transmission.

8. Method according to any of the claims 2-7, wherein when the conformity between the presence of the portable direct radiographic panel in the radiographic exposure stand with the radiographic exposure stand as specified in the radiological worklist is not established, a warning is given to the radiographic operator.

9. Method according to any of the preceding claims, wherein upon the radiographic exposure, the radiographic image and metadata stored in the direct radiographic panel are transmitted to the medical imaging system.

10. Medical imaging system comprising one or more radiographic exposure stands and one or more portable direct radiographic panels, comprising:
○ NFC tags provided on each radiographic exposure stand and on each portable direct radiographic panel;
○ means for positioning the radiographic panel in the radiographic exposure stand such that the NFC tag of the panel and the NFC tag of the stand are in each other's operating range;
○ means for detecting the presence of the panel in the radiographic exposure stand by a communication between the NFC tag of the panel and the NFC tag of the radiographic exposure;
○ means for communicating the presence of the radiographic panel in the radiographic exposure stand to the medical imaging system.

11. Medical imaging system according to claim 10 further comprising means for controlling whether for a radiographic exposure for which the exposure conditions are set forth in a radiological worklist, the radiographic exposure stand wherein the presence of the radiographic panel is detected, conforms to the radiographic exposure stand specified in said radiological worklist.

12. Medical imaging system according to claim 10 further comprising means for selecting, when the presence of a radiographic panel in more than one radiographic exposure stand is detected and the radiological exposure conditions are set forth in a radiological worklist, the radiographic exposure stand specified in said radiological worklist for the radiographic exposure, provided the presence of a radiographic panel in the selected exposure stand has been detected.

13. Medical imaging system according to any of the claims 10-12, wherein the NFC tag provided on each portable direct radiographic panel is an active tag, and the NFC tag provided on each radiographic exposure unit is a passive tag.

14. Medical imaging system according to any of the claims 8 to 10, comprising a radiographic workstation and wherein the portable direct radiographic panel and the workstation further comprise means for wireless communication by means of short-range radio waves, preferably through WIFI.

15. Medical imaging system according to any of the claims 10-14, comprising a radiological workstation provided with means for
○ receiving the communication of the presence of the radiographic panel in a radiographic exposure stand, and
○ performing the control set forth in claim 11 or the selection set forth in claim 12.

16. Medical imaging system according to any of the claims 10-15, wherein each radiographic exposure unit comprises at least two NFC tags for determining, apart from the presence, the orientation of the radiographic panel in the radiographic exposure unit.

17. Medical imaging system according to any of the claims 10-16, wherein the NFC tags of the radiographic exposure unit are installed on the bucky of such unit.
